# FASCICULE DE BREVET EUROPEEN

(11) **EP 0 625 334 B1**
(45) Date de publication et mention de la délivrance du brevet: **28.07.1999**
(21) Numéro de dépôt: 94401114.7
(22) Date de dépôt: 19.05.1994
(51) Int. Cl.: A61B 17/00, A61F 2/00

(54) **Ensemble de mise en place de prothèse en tissu souple par endoscopie**
Endoskopische Befestigungseinrichtung für Prothese aus Weichgewebe
Endoscopic installation assembly for soft tissue prosthesis

(30) Priorité: 21.05.1993 FR 9306104; 27.09.1993 FR 9311446
(43) Date de publication de la demande: 23.11.1994
(73) Titulaire: ETHICON, 92130 Issy les Moulineaux (FR)
(72) Inventeur: Bilweis, Joseph, F-78590 Noisy le Roi (FR); Arnaud, Axel, F-92300 Levallois Perret (FR); Catteau, Gilles, F-28630 Barjouville (FR); Prou, Philippe, F-28300 Saint Prest (FR)
(74) Mandataire: Martin, Jean-Jacques

(56) Documents cités:
- EP-A- 0 535 506
- WO-A-92/06638
- BE-A- 689 256
- FR-A- 980 853
- US-A- 3 115 360
- US-A- 4 161 365
- US-A- 5 036 854

## Description

La présente invention concerne le domaine des instruments pour chirurgie endoscopique.

La présente invention concerne plus précisément les instruments pour chirurgie endoscopique adaptés pour la mise en place de prothèses formées de tissu souple.

La chirurgie endoscopique englobe toutes les opérations qui sont réalisées en introduisant les instruments dans l'organisme par les voies naturelles ou par de minuscules incisions et avec examen de l'intérieur du corps à l'aide d'un appareil optique introduit dans l'organisme par les mêmes moyens.

On sait que la chirurgie endoscopique offre de nombreux avantages par rapport aux techniques chirurgicales classiques selon lesquelles le chirurgien opère à corps ouvert.

On peut en particulier citer les avantages suivants :
- la chirurgie endoscopique supprime les cicatrices,
- elle est plus confortable pour le patient et entraîne moins de douleur,
- elle conduit à une plus faible consommation de médicaments d'appoint, notamment des médicaments antalgiques-antidouleurs,
- elle permet une cicatrisation plus rapide,
- elle autorise un rétablissement plus rapide et par conséquent des séjours hospitaliers plus courts, notamment du fait que les complications opératoires et post-opératoires liées à l'ouverture du corps dans les techniques classiques, disparaissent.

Dans ces conditions, on estime ajourd'hui que dans les dix ans qui viennent, les 3/4 des interventions gynécologiques ou liées au système digestif seront réalisées par chirurgie endoscopique.

De nombreux instruments miniaturisés ont été proposés à cet effet, et en particulier des pinces, ciseaux, palpateurs, bistouris électriques ... Tous ces instruments miniaturisés sont conçus pour être introduits dans le corps, par la voie de trocarts de faible section.

Dans le domaine particulier de la mise en place de prothèses formées de tissu souple, les dispositifs jusqu'ici proposés ne donnent pas totalement satisfaction.

On a par exemple décrit dans le document EP-A-0 535 506 un système pour l'application de tissu anti-adhérentiel par voie endoscopique. Ce système comprend un support de tissu comprenant une zone de préhension de tissu, par exemple sous forme d'une fente longitudinale ou pince, et un tube applicateur cylindrique adapté pour recevoir un tissu enroulé sur le support et pour être engagé dans un trocart. Avant application le tissu doit être enroulé sur le support par rotation de celui-ci autour de son axe, puis l'ensemble ainsi formé doit être engagé dans le tube applicateur. Cette opération est assez délicate et difficile à réaliser dans des conditions aseptiques satisfaisantes. Le tissu est ensuite libéré et mis en place grâce à des pinces engagées par d'autres voies d'accès.

Le but de la présente invention est de perfectionner les dispositifs connus.

Ce but est atteint dans le cadre de la présente invention grâce à un ensemble de mise en place d'une prothèse formée d'un tissu souple, du type défini en revendication 1 annexée.

Comme on le précisera par la suite l'ensemble conforme à la présente invention offre de nombreux avantages par rapport aux dispositifs antérieurs connus.

Tout d'abord l'ensemble conforme à la présente invention permet un enroulement et une mise en place rapides de la prothèse, alors que les dispositifs antérieurs exigeaient une mise en oeuvre longue et délicate.

Ensuite, l'ensemble conforme à la présente invention permet une mise en oeuvre parfaitement aseptique, alors que les dispositifs antérieures laissaient à désirer sur ce point et généraient parfois des foyers d'infection.

Enfin, l'ensemble conforme à la présente invention permet d'enrouler la prothèse au tout dernier moment, avant son engagement dans un trocart en vue de son implantation, ce qui évite toute mémorisation de cette forme enroulée, comme on le rencontre pour des prothèses stockées longuement à l'état enroulé avant utilisation.

La présente invention a également pour but de faciliter la manipulation de l'ensemble précité et tout particulièrement de faciliter l'enroulement de la prothèse au moment de l'intervention chirurgicale.

Un autre but important de la présente invention est de permettre une manipulation de l'ensemble de mise en place précité tout en évitant tout contact avec la prothèse, y compris par l'intermédiaire de gants, avant son utilisation.

Ces derniers buts sont atteints dans le cadre de la présente invention grâce à un ensemble de mise en place du type précité comprenant en outre une plaquette thermoformée définissant une chambre sensiblement étanche apte à recevoir la prothèse, à plat, et un logement débouchant dans ladite chambre, apte à recevoir le fourreau et la fourche en position d'engagement.

Selon une autre caractéristique avantageuse de la présente invention, l'ensemble comprend également une fourche de mise en place comportant deux branches sollicitées élastiquement en éloignement.

La présente invention concerne préférablement un kit regroupant, sous emballage stérile, un fourreau tubulaire à fente, une prothèse et une fourche d'enroulement.

D'autres caractéristiques, buts et avantages de la présente invention apparaîtront à la lecture de la description détaillée qui va suivre, et en regard des dessins annexés donnés à titre d'exemple non limitatif et sur lesquels :
- la figure 1 représente une vue schématique en perspective d'un fourreau tubulaire à fente associé à une prothèse,
- la figure 2 représente une fourche d'enroulement correspondante conforme à un premier mode de réalisation de la présente invention,
- les figures 3 et 4 illustrent schématiquement la phase d'enroulement de la prothèse, à l'aide de la fourche, dans le fourreau tubulaire,
- la figure 5 illustre schématiquement le kit regroupant, sous emballage stérile, un fourreau tubulaire à fente, une prothèse et une fourche d'enroulement,
- la figure 6 représente une fourche de mise en place conforme à la présente invention,
- la figure 7 représente le fourreau tubulaire associé,
- la figure 8 représente la fourche de mise en place introduite dans le fourreau tubulaire,
- la figure 9 illustre schématiquement la phase de mise en place de la prothèse à l'aide de la fourche précitée, et
- la figure 10 représente une vue schématique en perspective d'un ensemble de mise en place conforme à la présente invention comprenant une plaquette therformée.

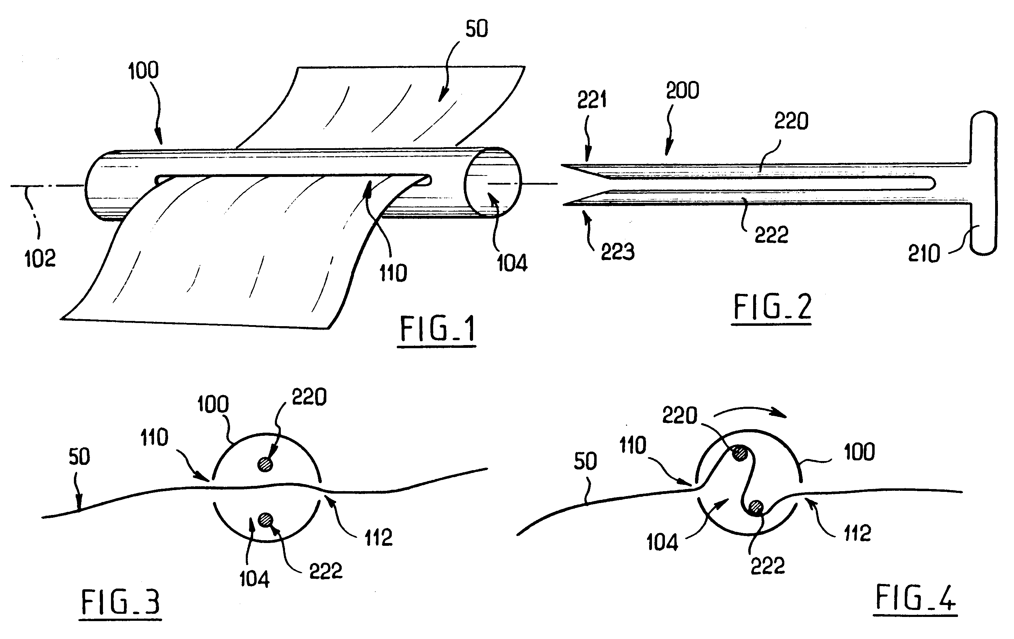

On va tout d'abord décrire l'ensemble de base de la présente invention comprenant un fourreau tubulaire 100 et une fourche d'enroulement 200 comme représenté sur les figures 1 à 5.

Le fourreau tubulaire 100 est réalisé en tout matériau approprié stérilisable, notamment en matière plastique ou métal.

Le fourreau tubulaire 100, centré sur un axe 102, comprend au moins une fente, de préférence deux fentes longitudinales 110, 112. Lesdites fentes 110, 112 sont de préférence diamétralement opposées. Elles sont avantageusement rectilignes et parallèles à l'axe 102.

Lesdites fentes longitudinales 110, 112 sont adaptées pour recevoir la prothèse 50 formée d'un tissu souple. La prothèse 50 peut être formée de tout matériau approprié connu de l'homme de l'art. Il s'agit par exemple, mais non limitativement, de prothèses 50 destinées au traitement d'hernies.

La géométrie de la prothèse 50 peut également faire l'objet de nombreuses variantes de réalisation. Selon le mode de réalisation non limitatif représenté sur les figures, la prothèse 50 présente un contour rectangulaire. L'invention n'est cependant pas limitée à cette géométrie particulière. Elle s'applique également par exemple à la mise en place de prothèse 50 de contour circulaire.

Il est important que la longueur des fentes 110, 112 soit au moins légèrement supérieure à l'une des dimensions de la prothèse 50. La prothèse 50 de contour rectangulaire peut présenter typiquement des dimensions de 10x12cm.

Comme on le voit sur la figure 1, lorsque le fourreau tubulaire 100 est positionné sur la prothèse 50, celle-ci est engagée dans les fentes 110, 112 et traverse par conséquent le volume intérieur 104 du fourreau 100 en émergeant sur l'extérieur du fourreau 100 respectivement par chacune des fentes 110, 112.

La fourche d'enroulement 200 est conçue pour être engagée dans le fourreau 100 respectivement de part et d'autre de la prothèse 50 et permettre l'enroulement de celle-ci dans le fourreau 100, par rotation autour de l'axe 102, comme représenté notamment sur les figures 3 et 4.

La fourche d'enroulement 200 peut faire l'objet de nombreuses variantes de réalisation. Elle est réalisée également en tout matériau approprié stérilisable, tel que notamment en matière plastique ou métal.

Selon le mode de réalisation particulier représenté sur la figure 2, la fourche d'enroulement 200 comprend une base 210 pourvue de deux branches rectilignes parallèles entre elles 220, 222. On notera que de préférence, les extrémités libres 221, 223 des branches 220, 222 sont effilées pour faciliter l'insertion de la fourche d'enroulement 200 dans le fourreau 100.

On a illustré sur la figure 3, la phase d'engagement de la fourche 200 dans le fourreau 100 respectivement de part et d'autre de la prothèse 50. En d'autres termes, lors de cette phase d'engagement les deux branches 220, 222 sont placées sur des côtés opposés de la prothèse 50, dans le volume interne 104 du fourreau 100. Il suffit alors d'entraîner la fourche 200 à rotation, dans un sens ou dans l'autre, autour de l'axe 102, pour enrouler la prothèse 50 à l'intérieur du fourreau 100, autour desdites branches 220, 222 de la fourche 200.

On notera qu'au cours de cette phase d'enroulement, on réalise plus précisément deux bobinages de la prothèse 50 sur elle-même à l'aide respectivement des deux branches 220, 222.

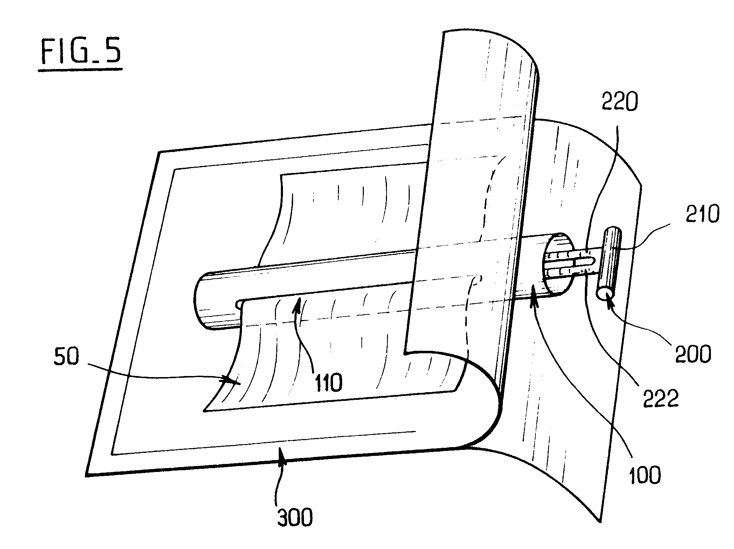

On a illustré sur la figure 5, un kit d'emballage stérile 300 recevant un fourreau tubulaire 100, une prothèse 50 et une fourche d'enroulement 200 comme décrits précédemment.

L'homme de l'art comprendra aisément qu'après ouverture de l'emballage 300 comme illustré sur la figure 5, un opérateur peut procéder de façon aseptique au bobinage du tissu souple 50 par rotation de la fourche d'enroulement 200 sous couvert du sachet pelable 300.

Une fois enroulée à l'intérieur du fourreau 100, comme décrit précédemment, la prothèse 50 peut être mise en place dans le corps par tout moyen adéquat tenant lieu de poussoir propre à éjecter ladite prothèse à l'extérieur du fourreau 100. Le cas échéant, la prothèse 50 peut être mise en place à l'aide de la fourche d'enroulement 200 elle-même, sous réserve que celle-ci soit adaptée, notamment quant à son embase 210, pour traverser le fourreau 100 d'une part, et de préférence pour être fixée à l'extrémité d'une tige applicateur d'autre part.

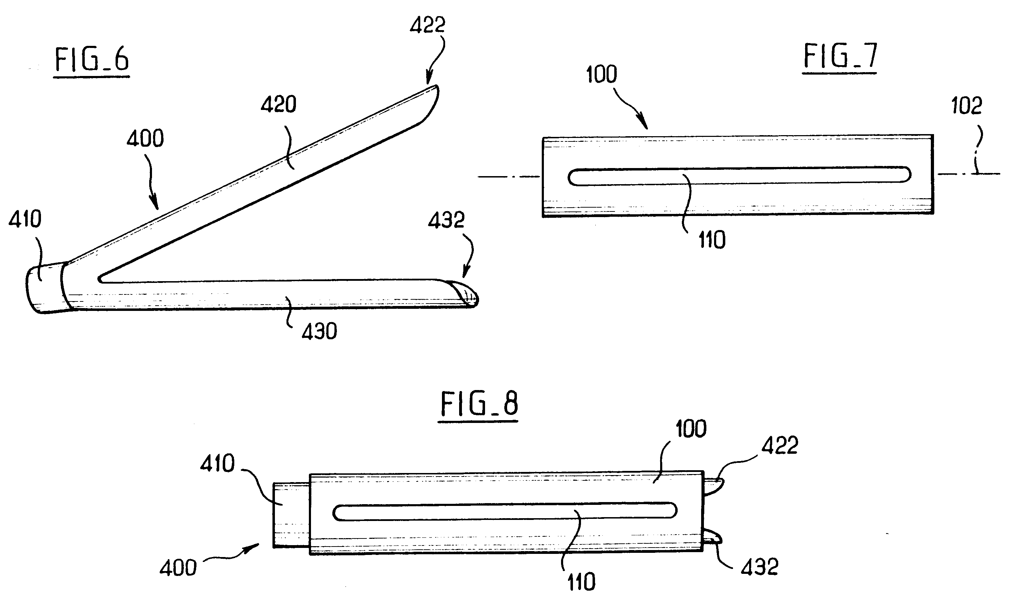

Toutefois, de préférence, la fourche d'enroulement 200 précitée est à usage unique et jetée après la phase d'enroulement précitée afin d'être remplacée, lors de la mise en place, par une fourche de mise en place 400 du type représenté sur les figures 6 et suivantes.

Cette fourche de mise en place 400 comporte une base 410 pourvue de deux branches 420, 430 sollicitées élastiquement en éloignement.

De façon comparable à la fourche d'enroulement 200, de préférence les extrémités libres 422, 432 des branches 420, 430 de la fourche de mise en place 400 sont effilées pour faciliter l'engagement de la fourche de mise en place 400 dans le fourreau 100.

De préférence, les deux branches 420, 430 sont rectilignes. La fourche 400 de mise en place peut comprendre une fourche rigide 430 et une fourche élastique 420 sollicitée en éloignement de cette dernière, ou encore deux branches 420, 430 sollicitées en éloignement l'une de l'autre de façon symétrique par rapport à l'axe de la base 410.

Là encore la fourche de mise en place 400 est réalisée en tout matériau approprié stérilisable, tel que notamment en matière plastique ou métal.

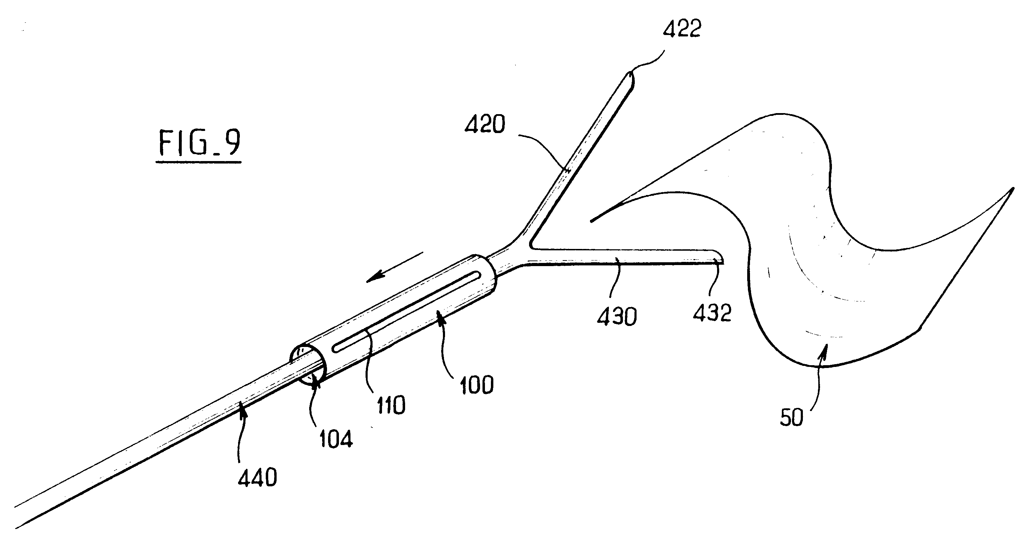

La fourche de mise en place 400, y compris sa base 410 est adaptée pour traverser le fourreau tubulaire 100 comme on le voit notamment sur les figures 8 et 9.

Ainsi, lorsque la fourche de mise en place 400 est engagée sur la prothèse 50 enroulée dans le fourreau 100 et translatée par rapport au fourreau 100 pour traverser celui-ci, comme représenté sur la figure 9, la prothèse 50 est libérée automatiquement dès que la fourche de mise en place 400 émerge quasi-totalement du fourreau 100. Il est à noter que le double enroulement de la prothèse 50 à l'intérieur du fourreau 100 facilite son déploiement ultérieur.

En outre, une fois déployée, la fourche de mise en place 400 peut être utilisée en tant qu'applicateur pour faciliter la manipulation et le positionnement de ladite prothèse 50, voire en tant qu'écarteur pour déplacer, si nécessaire, les tissus environnants du corps recevant la prothèse.

Pour permettre une translation relative du fourreau tubulaire 100 et de la fourche de mise en place 400 sous endoscopie, à l'intérieur du corps, l'embase 410 de la fourche de mise en place et le fourreau tubulaire 100 sont de préférence adaptés pour être fixés sur une extrémité respective de deux tiges applicatrices conçues pour être engagées dans un trocart commun. Sur la figure 9, on a illustré uniquement la tige applicatrice 440 associée à la fourche de mise en place 400. La tige applicatrice associée au fourreau tubulaire 100 n'est pas représentée pour simplifier l'illustration. En pratique, les deux tiges applicatrices peuvent être parallèles entre elles dans le trocart d'accès, voire concentriques entre elles.

Par ailleurs, on a indiqué précédemment que de préférence l'enroulement de la prothèse 50 à l'intérieur du fourreau 100 était réalisé à l'aide d'une fourche à usage unique spécifique 200 stockée de façon stérile dans l'emballage 300 avec le fourreau 100 et la prothèse 50, laquelle fourche d'enroulement 200 était jetée après enroulement et remplacée par la fourche de mise en place 400.

En variante cependant, on peut prévoir d'utiliser la fourche 400 à branches élastiques lors de la phase d'enroulement de la prothèse 50. Dans ce cas, la fourche d'enroulement 200 et la fourche de mise en place 400, sont confondues.

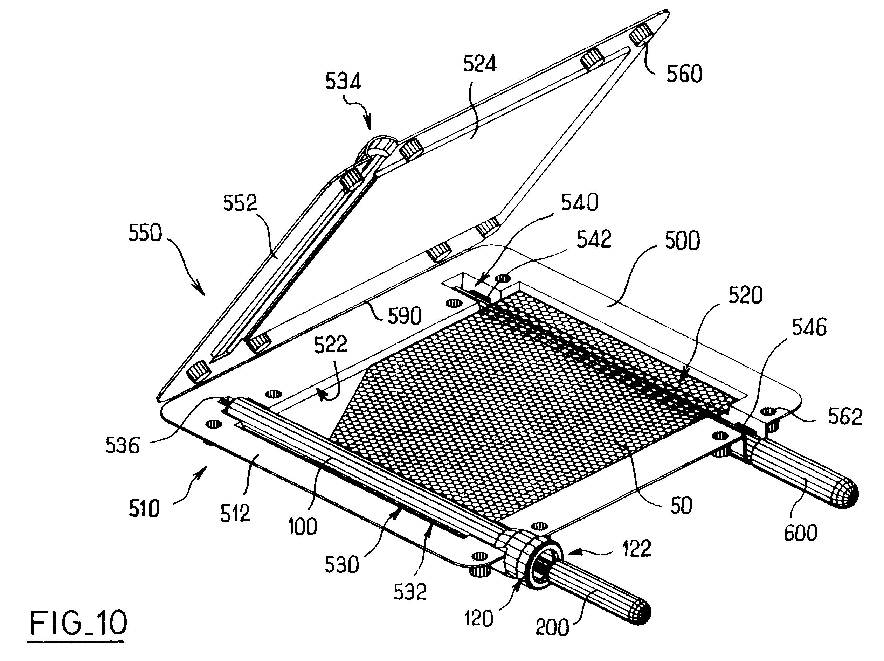

On va maintenant décrire la variante de réalisation conforme à la présente invention représentée sur la figure 10 annexée.

On aperçoit sur la figure 10 annexée un ensemble de mise en place d'une prothèse 50 formée d'un tissu souple, comprenant un fourreau tubulaire 100, une fourche d'enroulement 200 et une plaquette thermoformée 500.

La plaquette 500 conforme à la présente invention est de préférence formée à l'aide d'une feuille en matériau thermoplastique thermoformée.

Selon le mode de réalisation préférentiel représenté sur la figure 10, la plaquette 500 comprend une embase 510 et un couvercle 550 articulés entre eux le long de l'un de leurs bords rectilignes respectifs, au niveau d'une zone de liaison 590 formant charnière.

L'embase 510 et le couvercle 550 sont conformés pour définir en combinaison au moins une chambre 520 apte à recevoir la prothèse 50 à plat, comme on le voit sur la figure 10, et un logement 530 qui débouche dans ladite chambre 520 et conçu pour recevoir le fourreau 100 et la fourche 200 en position d'engagement.

Pour cela, diverses configurations de l'embase 510 et du couvercle 550 peuvent être admises. Le cas échéant, l'un de l'embase 510 et du couvercle 550 peut être formé d'un volet parfaitement plan tandis que la chambre 520 et/ou le logement 530 serait(ent) formé(s), sous forme de cavités thermoformées dans l'autre du couvercle 550 et de l'embase 510.

Toutefois, selon le mode de réalisation préférentiel représenté sur la figure 10 annexée, ladite chambre 520 ainsi que le logement 530 sont définis par des structures complémentaires réalisées à la fois sur l'embase 510 et sur le couvercle 550.

Ainsi, selon le mode de réalisation préférentiel représenté sur la figure 10, l'embase 510 possède une cavité 522 de contour adapté à la prothèse 50, soit avantageusement de contour rectangulaire.

La profondeur de cette cavité 522 est supérieure à l'épaisseur de la chambre 520 recherchée. La profondeur de la cavité 522 est avantageusement constante.

Par ailleurs, le couvercle 550 possède une saillie 524, de même contour que la cavité 522, mais de hauteur inférieure à la profondeur de cette dernière.

L'homme de l'art comprendra aisément que, lorsque le couvercle 550 repose sur l'embase 510, la saillie 524 pénètre dans la cavité 522, et la chambre 520 est ainsi définie entre les surfaces de base de la cavité 522 et de la saillie 524. L'épaisseur de la chambre 520 est ainsi définie par la différence entre la profondeur de la cavité 522 et la hauteur de la saillie 524, celle-ci étant avantageusement constante sur toute l'étendue de la saillie 524.

Il faut noter que de préférence la cavité 522 ainsi que la saillie 524, et par conséquent la chambre 520, sont entourées totalement par une bordure coplanaire 512, 552 formée respectivement sur l'embase 510 et sur le couvercle 550, de sorte que lorsque le couvercle 550 repose sur l'embase 510, ladite chambre 520 soit au moins sensiblement étanche.

De plus, l'embase 510 possède un canal 532 définissant le logement 530 pour le fourreau 100 et la fourche 200. Ce canal 532 débouche dans la cavité 522. Le canal 532 a de préférence une géométrie hémicylindrique. Le canal 532 s'étend parallèlement à l'un des bords de la cavité 522, de préférence perpendiculairement à la zone de charnière 590.

Par ailleurs, le canal 532 est avantageusement prévu en position adjacente à l'un des bords de ladite cavité 522, comme on le voit sur la figure 10.

Ainsi, avant enroulement, la prothèse 50 n'est pas disposée de part et d'autre du fourreau 100, mais seulement d'un côté de celui-ci. On comprend que dans ces conditions, la rotation relative de la fourche 200 et du fourreau 100 entraîne l'enroulement de la prothèse 50 dans le fourreau 100, dans un sens de rotation unique.

Plus précisément, le canal 532 est de préférence adapté pour entourer le fourreau 100 sur un secteur supérieur à 180° de sorte que le fourreau 100 émerge par rapport à la bordure coplanaire 512 de l'embase 510.

Dans ce cas, le logement 530 est défini, en complément du canal 532 précité par une calotte 534 complémentaire formée dans le couvercle 550.

La calotte 534 est définie par une enveloppe de contour cylindrique, rectiligne, perpendiculaire de préférence à la zone de charnière 590.

Le canal 532 et la calotte 534 définissent en combinaison le logement 530 cylindrique apte à recevoir le fourreau 100.

De préférence, le canal 532 est conçu pour retenir, à l'encontre d'un retrait intempestif, le fourreau 100. Pour cela le canal 532 peut posséder des bords convergents, ou encore on peut prévoir, notamment sur les extrémités du canal 532, des saillies thermoformées aptes à retenir ledit fourreau 100.

Plus précisément, comme on le voit sur la figure 10, la longueur du logement 530, soit du canal 532 et de la calotte 534, est supérieure à la longueur correspondante de la cavité 522, soit de la chambre 520.

Ainsi, le fourreau 100 est maintenu fermement par la plaquette 500, par ses deux extrémités, sur l'extérieur de ladite chambre 520.

Il faut noter cependant que pour permettre l'accès au fourreau 100 et à la fourche 200, pour entraîner ceux-ci à rotation relative, le logement 530 débouche d'un côté, sur l'extérieur de la plaquette 500.

De préférence, le logement 530 débouche sur l'extérieur de la plaquette 500, sur le bord de celle-ci opposé à la zone des charnière 590.

Selon une autre caractéristique avantageuse de l'invention, le logement 530 possède à son extrémité opposée à celle émergeant sur l'extérieur de la plaquette 500, une forme concave 536 conçue pour recevoir et guider à rotation les extrémités libres 221, 223 des branches 220, 222 de la fourche 200. Cette forme de guidage 536 permet de réaliser la fourche 200 sous forme de branches 220, 222 fines, tout en garantissant un parfait enroulement de la prothèse 50.

Selon le mode de réalisation préférentielle représenté sur la figure 10, l'embase 510 possède en outre un second canal 540 conçu pour recevoir un poussoir 600.

Le poussoir 600 est conçu pour être engagé dans le fourreau 100 après retrait de la fourche 200, et après enroulement de la prothèse 50, pour permettre l'expulsion de celle-ci sur le site d'utilisation.

Un tel poussoir 600 peut être formé d'un simple barreau de section, préférentiellement cylindrique, complémentaire de la chambre interne du fourreau 100. Ce poussoir 600 est destiné à compléter ou remplacer la fourche de mise en place 400 décrite précédemment.

Le canal 540 peut être séparé de la chambre 520. Toutefois, de préférence, le canal 540 débouche dans ladite chambre 520 afin de faciliter les opérations de stérilisation de l'ensemble.

Selon le mode de réalisation particulier représenté sur la figure 10, le second canal 540 est formé dans l'embase 510 sous la chambre 520. Le canal 540 est rectiligne et parallèle au second bord de l'embase 510 orthogonal à la zone de charnière 590.

Pour permettre l'accès au poussoir 600, le second canal 540 débouche également sur l'extérieur de la plaquette 500, sur le bord de celle-ci opposé à la zone de charnière 590.

Par ailleurs, de préférence, le second canal 540 est également adapté pour maintenir à l'encontre d'un retrait intempestif le poussoir 600. Pour cela, le canal 540 peut être formé à bords convergents pour entourer le poussoir 600 sur un secteur de sa périphérie supérieure à 180°, ou encore le second canal 540 peut être pourvu de dentures en saillie telles que référencées en 542 et 546, pour maintenir le poussoir 600.

Ces saillies 542, 546 sont formées dans les portions du canal 540 disposées à l'extérieur de la chambre 520.

De préférence, la plaquette 500 est pourvue de moyens permettant de fixer le couvercle 550 sur l'embase 510 après introduction de la prothèse 50. Ces moyens de fixation peuvent être formés de tout moyen connu approprié, tels que par exemple de moyens de collage ou de soudure aux ultrasons. Toutefois, de préférence et de façon connue en soi, le couvercle 550 est maintenu sur l'embase 510 grâce à des séries de formes d'encliquetage complémentaires prévues respectivement sur le couvercle 550 et l'embase 510, telles que par exemple des bossages, de préférence évasés, 560 formés sur le couvercle et adaptés pour pénétrer dans des cavités complémentaires 562 formées dans l'embase 510.

Selon la figure 10, il est prévu ainsi 4 paires de bossages 560/cavités 562 respectivement au voisinage des angles de la plaquette 500, soit sur chaque extrémité d'un canal 530 ou 540.

Il faut noter que selon une autre caractéristique avantageuse de l'invention, la tête 120 du fourreau 100 accessible sur l'extérieur de la plaquette 500 est évasée pour recevoir un joint ou valve 122. Ce joint 122 est conçu pour assurer la fermeture étanche du fourreau 100 lors de l'engagement et/ou du retrait de la fourche 200 et/ou du poussoir 600. Il est en effet important, lors d'interventions chirurgicales sous endoscopie, de veiller à assurer l'étanchéité au niveau du trocart d'intervention.

Une telle valve 122 peut être formée de toute structure classique connue de l'homme de l'art dans le domaine de l'endoscopie, tel que par exemple d'une valve à lèvres élastiques, en matériau élastomère. De telles valves à lèvres sont formées le plus souvent d'un ensemble de lèvres complémentaires réunies à une bague support par leur périphérie extérieure, et accolées deux à deux selon des rayons de cette bague.

De préférence, les diverses pièces composant l'ensemble de mise en place conforme à la présente invention, sont réalisées en matière plastique acceptées dans le domaine médical et autorisant une stérilisation.

Le cas échéant, cependant, le fourreau 100, la fourche 200 et le poussoir 600 peuvent être réalisés en métal stérilisable.

En pratique, la plaquette thermoformée 500 représentée sur la figure 10 est conditionnée dans un emballage stérile, lui-même placé dans un emballage de protection, tel qu'un cartonnage.

Eventuellement l'ensemble de mise en place représenté sur la figure 10 peut être complété par une fourche de mise en place à branches élastiques, comme décrit précédemment. Si cela s'avère nécessaire, cette fourche de mise ne place additionnelle peut être livrée dans le même emballage stérile que la plaquette thermoformée 500, voire logée dans une chambre complémentaire formée additionnellement dans cette plaquette 500.

Pour l'essentiel, l'utilisation de la prothèse 50 à l'aide de l'ensemble représenté sur la figure 10 reste identique à celle décrite précédemment.

Au moment de l'utilisation, il suffit en effet d'entraîner à rotation la fourche 200 autour de son axe, par rapport au fourreau 100, pour enrouler la prothèse 50 dans le fourreau 100.

Le fourreau 100 peut ensuite être introduit dans un trocart d'intervention. Pour éjecter la prothèse 50 sur le site d'utilisation, il suffit d'introduire le poussoir 600 ou tout moyen équivalent dans le fourreau 100.

La présente invention offre de nombreux intérêts par rapport aux systèmes antérieurs connus. Elle permet un enroulement simple et rapide de la prothèse 50. En particulier, il faut noter que la plaquette 500 guide efficacement la prothèse 50 au cours de son enroulement. L'invention autorise un stockage à plat de la prothèse 50 ce qui évite tout effet de mémorisation d'une géométrie enroulée et facilite des interventions ultérieures.

Par ailleurs et surtout il faut noter que l'ensemble conforme à l'invention, permet une utilisation totalement aseptique.

Bien entendu la présente invention n'est pas limitée aux modes de réalisation particuliers qui viennent d'être décrits mais s'étend à toute variante conforme à son esprit défini dans les revendications.

Ainsi, par exemple, les tiges applicatrices précitées utilisées pour manipuler le fourreau 100 et la fourche de mise en place 400 peuvent être solidaires en permanence de ces derniers, ou adaptées pour être fixées de façon amovible sur ceux-ci, par tout moyen approprié, tel que par coopération par filetage, montage à baïonnette ou encliquetage.

On comprendra aisément que l'ensemble conforme à la présente invention offre des avantages décisifs par rapport au système décrit dans le document EP-A-0 535 506. En particulier le fourreau à fentes conforme à la présente invention permet d'enrouler le tissu sous emballage stérile, sans avoir à intervenir manuellement pour engager un rouleau préformé dans un tube applicateur comme celà est requis avec les moyens du document EP-A-0 535 506. Ensuite la fourche de mise en place à branches élastiques conforme à la présente invention permet de faciliter la libération et la mise en place du tissu sans exiger l'utilisation de pinces engagées par des voies d'accès additionnelles comme celà est requis avec les moyens du document EP-A-0 535 506.

## Revendications

1. Ensemble de mise en place d'une prothèse formée d'un tissu souple (50), ledit ensemble comprenant une fourche (200) conçue pour l'enroulement de la prothèse par rotation et un fourreau tubulaire (100) adapté pour recevoir la fourche (200) et la prothèse (50) enroulée, caractérisé par le fait que :
- le fourreau tubulaire (100) est pourvu d'au moins une fente longitudinale (110, 112) adaptée pour recevoir ladite prothèse (50), et
- la fourche d'enroulement (200) est conçue pour être engagée dans le fourreau (100) respectivement de part et d'autre de la prothèse (50) et permettre l'enroulement de celle-ci dans le fourreau (100) par rotation.

2. Ensemble selon la revendication 1, caractérisé par le fait qu'il comprend en outre une fourche de mise en place (400) comportant deux branches (420, 430) sollicitées élastiquement en éloignement.

3. Ensemble selon l'une des revendications 1 ou 2, caractérisé par le fait que le fourreau (100) comprend deux deux fentes longitudinales (110, 112) sensiblement rectilignes et diamétralement opposées.

4. Ensemble selon l'une des revendications 1 à 3, caractérisé par le fait que chaque fente longitudinale (110, 112) du fourreau tubulaire (100) a une longueur supérieure à l'une des dimensions de la prothèse (50).

5. Ensemble selon l'une des revendications 1 à 4, caractérisé par le fait que la fourche d'enroulement (200) possède deux branches rectilignes (220, 222) à extrémités effilées pour faciliter l'insertion de ladite fourche (200) dans le fourreau tubulaire (100).

6. Ensemble selon l'une des revendications 1 à 5 prises en combinaison avec la revendication 2, caractérisé par le fait que la fourche de mise en place comprend deux branches (420, 430) sollicitées élastiquement en éloignement de façon symétrique par rapport à une base (410).

7. Ensemble selon l'une des revendications 1 à 5 prises en combinaison avec la revendication 2, caractérisé par le fait que la fourche de mise en place (400) comprend une branche rigide (430) et une branche élastique (420).

8. Ensemble selon l'une des revendications 1 à 7, caractérisé par le fait qu'il est réalisé en une matière plastique ou un métal stérilisable.

9. Ensemble selon l'une des revendications 1 à 8, caractérisé par le fait que le fourreau tubulaire (100) est adapté pour être fixé de façon amovible sur l'extrémité d'une tige applicatrice conçue pour être engagée dans un trocart d'accès.

10. Ensemble selon l'une des revendications 1 à 8, caractérisé par le fait que le fourreau tubulaire (100) est solidaire de l'extrémité d'une tige applicatrice adaptée pour être engagée dans un trocart d'accès.

11. Ensemble selon l'une des revendications 1 à 10 prises en combinaison avec la revendication 2, caractérisé par le fait que la fourche de mise en place (400) est adaptée pour être fixée sur l'extrémité d'une tige applicatrice conçue pour être engagée dans un trocart d'accès.

12. Ensemble selon l'une des revendications 1 à 10 prises en combinaison avec la revendication 2, caractérisé par le fait que la fourche de mise en place (400) est solidaire de l'extrémité d'une tige applicatrice adaptée pour être engagée dans un trocart d'accès.

13. Ensemble selon l'une des revendications 1 à 12, caractérisé par le fait qu'il comprend en outre une plaquette thermoformée (500) définissant une chambre (520) sensiblement étanche apte à recevoir la prothèse (50) à plat et un logement (530) débouchant dans ladite chambre (520), apte à recevoir le fourreau (100) et la fourche (200) en position d'engagement.

14. Ensemble selon la revendication 13, caractérisé par le fait que la plaquette thermoformée (500) est composée d'une embase (510) et d'un couvercle (550) articulés au niveau d'une zone de charnière (590) formant zone de liaison.

15. Ensemble selon la revendication 14, caractérisé par le fait que la chambre (520) conçue pour recevoir la prothèse (50) est définie par une cavité (522) réalisée dans l'embase (510).

16. Ensemble selon l'une des revendications 14 ou 15, caractérisé par le fait que la chambre (520) conçue pour recevoir la prothèse (50) est définie par une cavité (522) réalisée dans l'embase (510) et par une saillie (524) de même contour mais de plus faible hauteur réalisée dans le couvercle (550).

17. Ensemble selon l'une des revendications 13 à 16, caractérisé par le fait que la chambre (520) apte à recevoir la prothèse (50) est définie par des formes réalisées dans une embase (510) et/ou un couvercle (550) de la plaquette et entourées de toute part par une bordure coplanaire (512, 552).

18. Ensemble selon l'une des revendications 13 à 17, caractérisé par le fait que le logement (530) s'étend parallèlement à l'un des bords de la chambre (520).

19. Ensemble selon l'une des revendications 13 à 18, caractérisé par le fait que le logement (530) est adjacent à l'un des bords de la chambre (520).

20. Ensemble selon l'une des revendications 13 à 19, caractérisé par le fait que le logement (530) est conçu pour retenir le fourreau (100) à l'encontre d'un retrait intempestif.

21. Ensemble selon l'une des revendications 13 à 20, caractérisé par le fait que le logement (530) entoure le fourreau (100) sur un secteur supérieur à 180°.

22. Ensemble selon l'une des revendications 13 à 21, caractérisé par le fait que le logement (530) possède des saillies aptes à maintenir le fourreau (100).

23. Ensemble selon l'une des revendications 13 à 22, caractérisé par le fait que la longueur du logement (530) est supérieure à l'une des dimensions de la chambre (520).

24. Ensemble selon l'une des revendications 13 à 23, caractérisé par le fait que le logement (530) débouche sur l'extérieur de la plaquette thermoformée (500).

25. Ensemble selon l'une des revendications 13 à 24, caractérisé par le fait que l'une des extrémités du logement (530) est pourvue d'une forme (536) apte à guider la fourche (200) à rotation autour de son axe longitudinal.

26. Ensemble selon l'une des revendications 13 à 25, caractérisé par le fait qu'il comprend en outre un poussoir (600) de section sensiblement complémentaire du volume interne du fourreau (100).

27. Ensemble selon la revendication 26, caractérisé par le fait que la plaquette thermoformée (500) possède en outre un second canal (540) apte à recevoir le poussoir (600).

28. Ensemble selon la revendication 27, caractérisé par le fait que le canal (540) de réception du poussoir (600) débouche dans la chambre (520).

29. Ensemble selon l'une des revendications 27 ou 28, caractérisé par le fait que le canal (540) recevant le poussoir (600) est adapté pour maintenir celui-ci à l'encontre d'un retrait intempestif, de préférence en entourant le poussoir sur un secteur supérieur à 180° ou encore à l'aide de saillies (142, 146).

30. Ensemble selon l'une des revendications 13 à 29, caractérisé par le fait qu'il comprend des moyens de fixation du couvercle (550) sur l'embase (510).

31. Ensemble selon la revendication 30, caractérisé par le fait que les moyens de fixation sont formés par des bossages (560) de préférence évasés et des cavités complémentaires (562) prévues respectivement sur le couvercle (550) et l'embase (510).

32. Ensemble selon l'une des revendications 13 à 31, caractérisé par le fait que le fourreau (100) est pourvu à son embouchure d'une valve d'étanchéité (122).

33. Ensemble selon la revendication 32, caractérisé par le fait que la valve d'étanchéité (122) est formée d'une série de lèvres en matériau élastomère élastique équi-réparties autour de l'axe du fourreau.

34. Ensemble selon l'une des revendications 1 à 33 caracterisé par le fait qu'il comprend en outre un emballage stérile (300) formant kit qui loge le fourreau tubulaire (100), la prothèse en tissu souple (50) et la fourche d'enroulement (200).

## Claims

1. A system for installing a prosthesis formed by a flexible cloth (50), said system comprising a fork (20) designed to wind the prosthesis by rotation, and a tubular sheath adapted to receive the fork (200) and the wound prothesis (50), said system being characterized by the fact that:
the tubular sheath (100) is provided with at least one longitudinal slot (110, 112) adapted to receive said prosthesis (50); and
the winding fork (200) is designed to be engaged in the sheath (100) on respective opposite sides of the prosthesis (50) and to enable it to be wound up inside the sheath (100) by rotation.

2. A system according to claim 1, characterized by the fact that it further includes an installing fork (400) having two branches (420, 430) that are resiliently urged apart.

3. A system according to claim 1 or 2, characterized by the fact that the sheath (100) includes two longitudinal slots (110, 112) substantially rectilinear and diametrically opposite.

4. A system according to any one of claims 1 to 3, characterized by the fact that each longitudinal slot (110, 112) of the tubular sheath (100) is longer than one of the dimensions of the prosthesis (50).

5. A system according to any one of claims 1 to 4, characterized by the fact that the winding fork (400) has two rectilinear branches (220, 222) with tapering ends to facilitate insertion of said fork (200) in the tubular sheath (100).

6. A system according to any one of claims 1 to 5, taken in combination with claim 2, characterized by the fact that the installing fork comprises two branches (420, 430) that are resiliently urged apart symmetrically relative to a base (410).

7. A system according to any one of claims 1 to 5 taken in combination with claim 2, characterized by the fact that the installing fork (400) comprises a rigid branch (430) and a resilient branch (420).

8. A system according to any one of claims 1 to 7, characterized by the fact that it is made of a sterilizable plastics material or of a sterilizable metal.

9. A system according to any one of claims 1 to 8, characterized by the fact that the tubular sheath (100) is adapted to be fixed in removable manner on the end of an applicator rod designed to be engaged in an access trocar.

10. A system according to any one of claims 1 to 8, characterized by the fact that the tubular sheath (100) is secured to the end of an applicator rod adapted to be engaged in an access trocar.

11. A system according to any one of claims 1 to 10, taken in combination with claim 2, characterized by the fact that the installing fork (400) is adapted to be fixed on the end of a known applicator rod suitable for being engaged in an access trocar.

12. A system according to any one of claims 1 to 10 taken in combination with claim 2, characterized by the fact that the installing fork (400) is secured to the end of an applicator rod adapted to be engaged in an access trocar.

13. A system according to any one of claims 1 to 12, characterized by the fact that it further comprises a thermoformed plate (500) defining a substantially sealed chamber (520) suitable for receiving the prosthesis (50) while flat, and a housing (530) opening out into said chamber (100) and suitable for receiving the sheath (100) and the fork (200) in the engagement position.

14. A system according to claim 13, characterized by the fact that the thermoformed plate (500) is constituted by a base (510) and a cover (550) that are hinged together at a hinge zone (590) forming a link zone.

15. A system according to claim 14, characterized by the fact that the chamber (520) designed to receive the prosthesis (50) is defined by a cavity (522) formed in the base (510).

16. A system according to claim 14 or 15, characterized by the fact that the chamber (520) designed to receive the prosthesis (50) is defined by a cavity (522) formed in the base (510), and by a projection (524) having the same outline but of smaller height and formed in the cover (550).

17. A system according to any one of claims 13 to 16, characterized by the fact that the chamber (520) suitable for receiving the prosthesis (50) is defined by shapes formed in a base (510) and/or in a cover (550) of the plate, and surrounded on all sides by a coplanar margin (512, 552).

18. A system according to any one of claims 13 to 17, characterized by the fact that the housing (530) extends parallel to one of the edges of the chamber (520).

19. A system according to any one of claims 13 to 18, characterized by the fact that the housing (530) is adjacent to one of the edges of the chamber (520).

20. A system according to any one of claims 13 to 19, characterized by the fact that the housing (530) is designed to retain the sheath (100) against untimely withdrawal.

21. A system according to any one of claims 13 to 20, characterized by the fact that the housing (530) surrounds the sheath (100) over a sector of more than 180°.

22. A system according to any one of claims 13 to 21, characterized by the fact that the housing (530) possesses projections suitable for holding the sheath (100).

23. A system according to any one of claims 13 to 22, characterized by the fact that the length of the housing (530) is greater than one of the dimensions of the chamber (520).

24. A system according to any one of claims 13 to 23, characterized by the fact that the housing (530) opens to the outside of the thermoformed plate (500).

25. A system according to any one of claims 13 to 24, characterized by the fact that one of the ends of the housing (530) is provided with a shape (536) suitable for guiding the fork (200) in rotation about its longitudinal axis.

26. A system according to any one of claims 13 to 25, characterized by the fact that it further comprises a section bar pusher (600) that is substantially complementary to the inside volume of the sheath (100).

27. A system according to claim 26, characterized by the fact that the thermoformed plate (500) also includes a second channel (540) suitable for receiving the pusher (600).

28. A system according to claim 27, characterized by the fact that the channel (540) for receiving the pusher (600) opens out into the chamber (520).

29. A system according to claim 27 or 28, characterized by the fact that the channel (540) receiving the pusher (600) is adapted to retain the pusher against untimely withdrawal thereof, preferably by surrounding the pusher over a sector of more than 180° or else with the help of projections (142, 146).

30. A system according to any one of claims 13 to 29, characterized by the fact that it includes fixing means for fixing the cover (550) on the base (510).

31. A system according to claim 30, characterized by the fact that the fixing means are formed by projections (560) that are preferably flared and by complementary cavities (562), the projections and the cavities being provided respectively on the cover (550) and on the base (510).

32. A system according to any one of claims 13 to 31, characterized by the fact that the sheath (100) is provided at its mouth with a sealing valve (122).

33. A system according to claim 32, characterized by the fact that the sealing valve (122) is constituted by a series of lips made of resilient elastomer material and uniformly distributed around the axis of the sheath.

34. A system according to any one of claims 1 to 33, characterized by the fact that it further comprises a sterile packaging (300) providing a kit which houses the tubular sheath (100), the flexible cloth prosthesis (50), and the winding fork (200).

## Patentansprüche

1. Befestigungsvorrichtung für eine aus einem Weichgewebe gebildete Prothese (50), wobei die Vorrichtung eine Gabel (200), welche für das Aufrollen der Prothese durch Drehung eingerichtet ist, und eine röhrenförmige Hülse (100) aufweist, welche dafür eingerichtet ist, die Gabel (200) und die aufgerollte Prothese (50) aufzunehmen, dadurch gekennzeichnet, daß:
- die röhrenförmige Hülse (100) mit wenigstens einem Längsschlitz (110, 112) versehen ist, welcher dafür eingerichtet ist, die Prothese (50) aufzunehmen, und
- die Aufrollgabel (200) dafür gebildet ist, in die Hülse (100) jeweils beidseits der Prothese (50) eingeschoben zu werden und das Aufrollen dieser in der Hülse (100) durch Drehung zu ermöglichen.

2. Vorrichtung gemäß Anspruch 1, dadurch gekennzeichnet, daß sie außerdem eine Befestigungsgabel (400) mit zwei Armen (420, 430) aufweist, welche in Abstand voneinander elastisch beansprucht sind.

3. Vorrichtung gemäß einem der Ansprüche 1 oder 2, dadurch gekennzeichnet, daß die Hülse (100) zwei praktisch geradlinige und diametral entgegengesetzte Längsschlitze (110, 112) aufweist.

4. Vorrichtung gemäß einem der Ansprüche 1 bis 3, dadurch gekennzeichnet, daß jeder Längsschlitz (110, 112) der röhrenförmigen Hülse (100) eine größere Länge als eine der Abmessungen der Prothese (50) hat.

5. Vorrichtung gemäß einem der Ansprüche 1 bis 4, dadurch gekennzeichnet, daß die Aufrollgabel (200) zwei geradlinige Arme (220, 222) mit verjüngten Enden besitzt, um das Einführen der Gabel (200) in die röhrenförmige Hülse (100) zu erleichtern.

6. Vorrichtung gemäß einem der Ansprüche 1 bis 5 in Kombination mit Anspruch 2, dadurch gekennzeichnet, daß die Befestigungsgabel zwei symmetrisch bezüglich einer Basis (410) beabstandete, elastisch beanspruchte Arme (420, 430) aufweist.

7. Vorrichtung gemäß einem der Ansprüche 1 bis 5 in Kombination mit Anspruch 2, dadurch gekennzeichnet, daß die Befestigungsgabel (400) einen steifen Arm (430) und einen elastischen Arm (420) aufweist.

8. Vorrichtung gemäß einem der Ansprüche 1 bis 7, dadurch gekennzeichnet, daß sie aus einem Kunststoffmaterial oder einem sterilisierbaren Material hergestellt ist.

9. Vorrichtung gemäß einem der Ansprüche 1 bis 8, dadurch gekennzeichnet, daß die röhrenförmige Hülse (100) dafür eingerichtet ist, in abnehmbarer Weise auf dem Ende einer Einsetzstange befestigt zu werden, welche gebildet ist, um in einen Zugangstrokar eingeführt zu werden.

10. Vorrichtung gemäß einem der Ansprüche 1 bis 8, dadurch gekennzeichnet, daß die röhrenförmige Hülse (100) einstückig mit dem Ende einer Einsetzstange ausgebildet ist, welche dafür eingerichtet ist, in einen Zugangstrokar eingeführt zu werden.

11. Vorrichtung gemäß einem der Ansprüche 1 bis 10 in Kombination mit Anspruch 2, dadurch gekennzeichnet, daß die Befestigungsgabel (400) dafür eingerichtet ist, auf dem Ende einer Einsetzstange befestigt zu werden, welche gebildet ist, um in einen Zugangstrokar eingeführt zu werden.

12. Vorrichtung gemäß einem der Ansprüche 1 bis 10 in Kombination mit Anspruch 2, dadurch gekennzeichnet, daß die Befestigungsgabel (400) einstückig mit dem Ende einer Einsetzstange ausgebildet ist, welche dafür eingerichtet ist, in einen Zugangstrokar eingeführt zu werden.

13. Vorrichtung gemäß einem der Ansprüche 1 bis 12, dadurch gekennzeichnet, daß sie außerdem eine thermogeformte Platte (500), welche eine praktisch dichte Kammer (520) definiert, welche dafür eingerichtet ist, die glattgezogene Prothese (50) aufzunehmen, und eine Aufnahme (530) aufweist, welche in die Kammer (520) mündet und dafür eingerichtet ist, die Hülse (100) und die Gabel (200) in eingegriffener Position aufzunehmen.

14. Vorrichtung gemäß Anspruch 13, dadurch gekennzeichnet, daß die thermogeformte Platte (500) von einer Befestigungsfläche (510) und einem Deckel (550) gebildet ist, welche auf der Ebene eines Scharnierbereiches (590), der einen Verbindungsbereich bildet, gelenkig aneinander angebracht sind.

15. Vorrichtung gemäß Anspruch 14, dadurch gekennzeichnet, daß die für die Aufnahme der Prothese (50) gebildete Kammer (520) von einer in der Befestigungsfläche (510) gebildeten Vertiefung (522) definiert ist.

16. Vorrichtung gemäß einem der Ansprüche 14 oder 15, dadurch gekennzeichnet, daß die für die Aufnahme der Prothese (50) gebildete Kammer (520) von einer in der Befestigungsfläche (510) gebildeten Vertiefung (522) und von einer in dem Deckel (550) gebildeten Ausstülpung (524) mit demselben Umriß, aber einer geringeren Höhe definiert ist.

17. Vorrichtung gemäß einem der Ansprüche 13 bis 16, dadurch gekennzeichnet, daß die für die Aufnahme der Prothese (50) geeignete Kammer (520) von in einer Befestigungsfläche (510) und/oder einem Deckel (550) der Platte gebildeten und überall von einer koplanaren Einfassung (512, 552) umgebenen Formen definiert ist.

18. Vorrichtung gemäß einem der Ansprüche 13 bis 17, dadurch gekennzeichnet, daß sich die Aufnahme (530) parallel zu einer der Kanten der Kammer (520) erstreckt.

19. Vorrichtung gemäß einem der Ansprüche 13 bis 18, dadurch gekennzeichnet, daß die Aufnahme (530) an eine der Kanten der Kammer (520) anstoßend angeordnet ist.

20. Vorrichtung gemäß einem der Ansprüche 13 bis 19, dadurch gekennzeichnet, daß die Aufnahme (530) dafür eingerichtet ist, die Hülse (100) entgegen einem ungewollten Zurückziehen festzuhalten.

21. Vorrichtung gemäß einem der Ansprüche 13 bis 20, dadurch gekennzeichnet, daß die Aufnahme (530) die Hülse (100) über ein größeres Bogenstück als 180° umgibt.

22. Vorrichtung gemäß einem der Ansprüche 13 bis 21, dadurch gekennzeichnet, daß die Aufnahme (530) Vorsprünge besitzt, welche dafür geeignet sind, die Hülse (100) festzuhalten.

23. Vorrichtung gemäß einem der Ansprüche 1 bis 22, dadurch gekennzeichnet, daß die Länge der Aufnahme (530) größer als eine der Abmessungen der Kammer (520) ist.

24. Vorrichtung gemäß einem der Ansprüche 13 bis 23, dadurch gekennzeichnet, daß die Aufnahme (530) auf die Außenfläche der thermogeformten Platte (500) mündet.

25. Vorrichtung gemäß einem der Ansprüche 13 bis 14, dadurch gekennzeichnet, daß eines der Endes der Aufnahme (530) mit einer Form (536) versehen ist, welche dafür geeignet ist, die Gabel (200) in Drehung um ihre Längsachse zu führen.

26. Vorrichtung gemäß einem der Ansprüche 13 bis 25, dadurch gekennzeichnet, daß sie außerdem einen Stößel (600) mit praktisch komplementärem Querschnitt zu dem Innenvolumen der Hülse (100) aufweist.

27. Vorrichtung gemäß Anspruch 26, dadurch gekennzeichnet, daß die thermogeformte Platte (500) außerdem einen zweiten Kanal (540) besitzt, welcher dafür geeignet ist, den Stößel (600) aufzunehmen.

28. Vorrichtung gemäß Anspruch 27, dadurch gekennzeichnet, daß der Aufnahmekanal (540) für den Stößel (600) in die Kammer (520) mündet.

29. Vorrichtung gemäß einem der Ansprüche 27 oder 28, dadurch gekennzeichnet, daß der den Stößel (600) aufnehmende Kanal (540) dafür eingerichtet ist, diesen entgegen eines ungewollten Zurückziehens festzuhalten, vorzugsweise indem er den Stößel auf einem größeren Bogenstück als 180° umgibt oder auch mit Hilfe von Vorsprüngen (142, 146).

30. Vorrichtung gemäß einem der Ansprüche 13 bis 29, dadurch gekennzeichnet, daß sie Befestigungseinrichtungen für den Deckel (550) auf der Befestigungsfläche (510) aufweist.

31. Vorrichtung gemäß Anspruch 30, dadurch gekennzeichnet, daß die Befestigungseinrichtungen von vorzugsweise konisch erweiterten Höckern (560) und jeweils auf dem Deckel (550) und der Befestigungsfläche (510) vorgesehenen komplementären Vertiefungen (562) gebildet sind.

32. Vorrichtung gemäß einem der Ansprüche 13 bis 31, dadurch gekennzeichnet, daß die Hülse (100) an ihrer Öffnung mit einem Dichtungsventil (122) versehen ist.

33. Vorrichtung gemäß Anspruch 32, dadurch gekennzeichnet, daß das Dichtungsventil (122) von einer Reihe Dichtlippen aus elastischem Elastomermaterial gebildet ist, welche gleichmäßig um die Achse der Hülse herum verteilt sind.

34. Vorrichtung gemäß einem der Ansprüche 1 bis 33, dadurch gekennzeichnet, daß sie außerdem eine sterile Hülle (300) aufweist, welche einen Kasten bildet, der die röhrenförmige Hülse (100), die Prothese aus Weichgewebe (50) und die Aufrollgabel (200) unterbringt.
